# EUROPÄISCHE PATENTSCHRIFT

(11) **EP 0 640 325 B1**
(45) Veröffentlichungstag und Bekanntmachung des Hinweises auf die Patenterteilung: **15.07.1998**
(21) Anmeldenummer: 93810618.4
(22) Anmeldetag: 30.08.1993
(51) Int. Cl.: A61F 2/34

(54) **Mehrteilige künstliche Gelenkpfanne und Hüftgelenkprothese mit einer derartigen Gelenkpfanne**
Multi-part artificial acetabular cup and hip joint prosthesis with such a cup
Coque acétabulaire artificielle en plusieurs parties et prothèse de l'articulation de la hanche avec une telle coque

(43) Veröffentlichungstag der Anmeldung: 01.03.1995
(73) Patentinhaber: Sulzer Orthopädie AG, 6340 Baar (CH)
(72) Erfinder: Spotorno, Lorenzo, Prof. Dr. med., I-17024 Finale Ligure (IT); Willi, Roland, CH-8413 Neftenbach (CH)
(74) Vertreter: Trieblnig, Adolf

(56) Entgegenhaltungen:
- EP-A- 0 169 978
- EP-A- 0 341 199
- EP-A- 0 420 795
- EP-A- 0 445 068
- EP-A- 0 482 320
- EP-B- 0 308 297
- WO-A-85/00284
- DE-A- 3 602 081
- DE-C- 3 341 723
- FR-A- 2 597 329
- FR-A- 2 645 433

## Beschreibung

Die Erfindung betrifft einen Gelenkpfanne entsprechend dem Oberbegriff des Patentanspruchs 1, sowie eine Hüftgelenkprothese mit einer derartigen Gelenkpfanne.

Eine bekannte Gelenkpfanne der genannten Art enthält eine Aussenschale mit einer geschlossenen, relativ starren äquatorialen Randpartie und mit um diese beweglichen Segmenten, welche mit gegen den Polbereich der Aussenschale weisenden, gegenüber dem Polbereich nach innen auslenkbaren freien Endpartien ausgeführt und in diesen Endpartien mit in das Knochengewebe eindrückbaren Spitzen versehen sind (FR-A-2 645 433). Bei dieser bekannten Ausführung werden die dem Polbereich zugekehrten Endpartien der Segmente beim Einsetzen der Aussenschale in den Implantationsbereich nach innen gedrängt und anschliessend, durch eine formschlüssig in die Aussenschale einsetzbare Zwischenschale nach aussen gedrängt und durch die in das Knochengewebe eindringenden Spitzen in dem an den Polbereich der Aussenschale anschliessenden Abschnitt des Implantationsbereichs verankert. Die Aussenschale, die Zwischenschale und die in diese einsetzbare Innenschale sind zu einem als Ganzes formstabilen Implantat Zusammensetzbar, welches eine relativ starre Verbindung zwischen der einen Gelenkkopf aufnehmenden Innenschale und dem Knochengewebe bildet. Es hat sich gezeigt, dass bei der Verwendung derartiger, relativ starrer Gelenkpfannen unter Belastung, z.B. aufgrund von elastischen Verformungen der die Aussenschale umgebenden Knochenpartie, etwa bei einem Aufweiten eines Teils des Implantationsbereichs, Teile des Knochengewebes sich örtlich von der Aussenschale abheben können, so dass im übrigen Teil des Implantationsbereichs entsprechende, relativ hohe örtliche Beanspruchungen des Knochengewebes auftreten können.

Der Erfindung liegt die Aufgabe zugrunde, eine insbesondere in dieser Hinsicht weiter entwickelte, verbesserte Gelenkpfanne zu schaffen, welche eine innerhalb vorbestimmter Grenzen nachgiebige Verbindung zwischen der Innenschale und dem Knochengewebe gewährleistet und durch welche auch bei elastischen Verformungen der die Gelenkpfanne aufnehmenden Knochenpartie eine im wesentliche gleichbleibende Beanspruchung des Knochengewebes erzielbar ist.

Diese Aufgabe wird gemäss der Erfindung durch die im kennzeichnenden Teil des Patentanspruchs 1 angegebenen Merkmale gelöst.

Die erfindungsgemässe Ausbildung der Gelenkpfanne gestattet eine Anpassung der in Segmente unterteilten äquatorialen Randpartie der Aussenschale an die bei Belastung auftretenden Verformungen der sie umgebenden Knochenpartie, so dass auch unter Beanspruchung durch wechselnde Kräfte eine kraft- und formschlüssige Verbindung zwischen jedem der relativ zueinander beweglichen Segmente der Aussenschale und dem Knochengewebe gewährleistet ist. Entsprechend kann ein örtliches Ablösen des Knochengewebes von einem Teil oder Teilen der Aussenschale vermieden und damit eine nachteilige ungleichmässige Beanspruchung des Knochengewebes durch örtlich auftretende erhöhte Druckkräfte verhindert werden. Durch das zwischen der Aussenschale und der Zwischenschale bestehende Spiel können sich die Zwischenschale und die Innenschale unter wechselnden Kräften entsprechend um das Befestigungselement hin und her bewegen, wobei die sphärische Form der Innenschale für die Führung des Gelenkkopfs erhalten bleibt.

In den abhängigen Patentansprüchen sind vorteilhafte Weiterbildungen des Erfindungsgegenstandes angegeben.

Weitere Einzelheiten und Merkmale ergeben sich aus der folgenden Beschreibung von in der Zeichnung schematisch dargestellten Ausführungsbeispielen nach der Erfindung, in Verbindung mit den Patentansprüchen. Es zeigen:
- Fig. 1: einen Teil einer Hüftgelenkprothese mit einer erfindungsgemäss ausgebildeten Gelenkpfanne in einem diametral verlaufenden Schnitt;
- Fig. 2: die Gelenkpfanne nach Fig. 1 in einer Draufsicht;
- Fig. 3: eine Gelenkpfanne in einem diametral verlaufenden Schnitt nach einer abgewandelten Ausführungsform;
- Fig. 4: die Gelenkpfanne nach Fig. 3 in einer Draufsicht;
- Fig. 5: eine Gelenkpfanne in einem diametral verlaufenden Schnitt nach einer weiteren Ausführungsform und
- Fig. 6: die Gelenkpfanne nach Fig. 5 in einer Draufsicht.

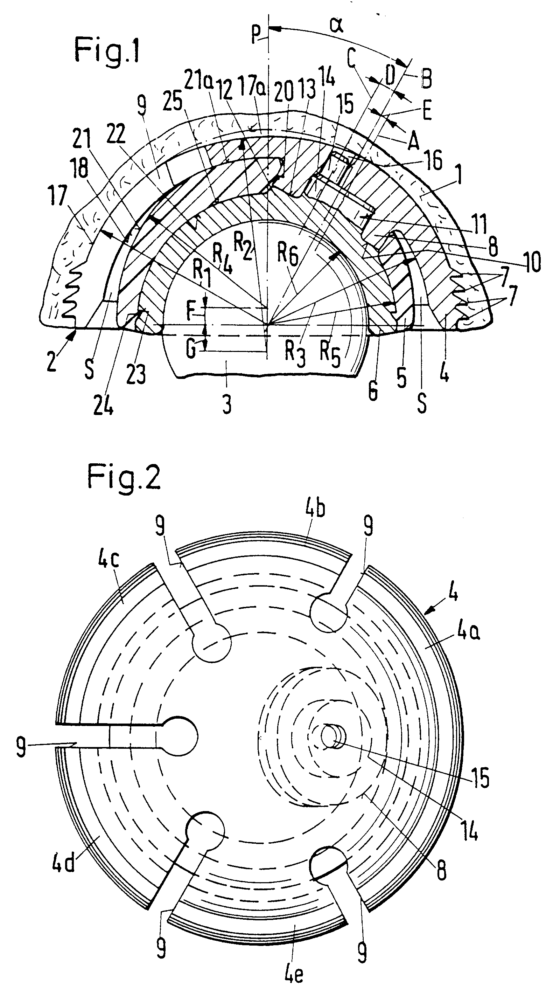

Die Hüftgelenkprothese nach den Fig. 1 und 2 enthält eine in das Knochengewebe 1 eines menschlichen Beckenteils einsetzbare mehrteilige Gelenkpfanne 2 und einen Gelenkkopf 3, der auf einem nicht dargestellten Schaftteil angeordnet ist, welcher in bekannter Weise in einem Röhrenknochen, beim vorliegenden Beispiel einem Oberschenkelknochen, befestigbar ist. Die Gelenkpfanne 2 enthält eine in eine vorbereitete Ausnehmung des Knochengewebes 1 einführbare und in dieser verankerbare metallische Aussenschale 4 im wesentlichen in Form einer hohlen Halbkugel, eine in diese einsetzbare, aus einem Kunststoff bestehende, ebenfalls im wesentlichen halbkugelförmige Zwischenschale 5 und eine in diese formschlüssig einsetzbare, im wesentlichen konzentrisch zur Aussenschale 4 positionierbare halbkugelförmige Innenschale 6, welche den Gelenkkopf 2 aufnimmt.

Die Aussenschale 4 ist durch in Meridianen verlaufende Schlitze 9, die sich aus ihrem äquatorialen Umfangsbereich gegen den Polbereich hin erstrecken, in mehrere, dargestellungsgemäss fünf, relativ zueinander bewegliche Segmente 4a, 4b, 4c, 4d, 4e unterteilt, welche dementsprechend unter Eigenspannung in den Implantationsbereich einsetzbar und gegen das Knochengewebe verspannbar sind. Die Aussenschale 4 ist ferner im äquatorialen Umfangsbereich mit mehreren, in Umfangsrichtung verlaufenden, in das Knochengewebe einführbaren bzw. eindrück- oder einschlagbaren zahnartigen Vorsprüngen 7 versehen, über welche die Segmente 4a, 4b, 4c, 4d, 4e in dem den Implantationsbereich umgebenden Knochengewebe 1 verankerbar sind. Die Aussenschale 4 und die Innenschale 6 sind in Richtung einer gemeinsamen, zumindest annähernd radialen Hauptachse A starr und bezüglich dieser Hauptache A drehfest miteinander verbunden. Dazu ist die Aussenschale 4 mit einer nach innen abstehenden Aufsetzpartie 8 ausgeführt, die eine der Innenschale 6 zugekehrte Aufsetzfläche 10 und eine nach innen offene Führungsbohrung 11 aufweist, während die Innenschale 6 mit einer Stützpartie 12 ausgeführt ist, die eine an die Aufsetzfläche 10 anlegbare Stützfläche 13 und einen von dieser nach aussen abstehenden, in die Führungsbohrung 11 einführbaren zapfenartigen Führungsteil 14 aufweist. Zur Sicherung der drehfesten Verbindung zwischen der Aussenschale 4 und der Innenschale 6 ist am Führungsteil 14 ein Befestigungselement, darstellungsgemäss in Form eines Zentrierstiftes 15, vorgesehen, welches mit einer gegenüber der Hauptachse A um ein Mass D exzentrisch versetzten Achse C angeordnet ist und welches in eine entsprechende, an der Aussenschale 4 vorgesehene Aussparung 16 einführbar ist.

Die gemeinsame Hauptachse A der Aussenschale 4 und der Innenschale 6 kann im wesentlichen mit einer durch die anatomischen Verhältnisse im Beckenbereich bestimmten Wirkungslinie einer über die Gelenkpfanne 2 zu übertragenden Hauptbelastungskraft B zusammenfallen oder, wie in Fig. 1 dargestellt, um ein z.B. konstruktiv bestimmtes Mass E gegenüber dieser Wirkungslinie versetzt sein, welche mit einer Polachse P der Aussenschale 4 einen Winkel α einschliesst. Die Aussenschale 4 ist darstellungsgemäss in einem Polbereich 17a mit einer geringeren Wandstärke ausgeführt als im äquatorialen Bereich und weist eine Aussenfläche 17 mit einer durch einen Radius R1 bestimmten sphärischen Hauptpartie und einer durch einen grösseren Radius R2 bestimmten, abgeflachten Partie im Polbereich 17a sowie eine durch einen Radius R3 bestimmte sphärische Innenfläche 18 auf. Die geringere Wandstärke der Aussenschale 4 im Polbereich 17a ermöglicht eine elastische Verformung der Segmente 4a, 4b, 4c, 4d, 4e in einem vorbestimmten Biegebereich und erleichtert dadurch das Einsetzen der Aussenschale 4 und deren Primärverankerung im Implantationsbereich.

Die Zwischenschale 5 ist mit einer zur Aufnahme der Aufsetzpartie 8 und der Stützpartie 12 geeigneten Durchtrittsöffnung 20 versehen sowie mit einer sphärischen Aussenfläche 21 und einer sphärischen Innenfläche 22 ausgeführt, deren Krümmungmittelpunkte darstellungsgemäss auf der Polachse P gegeneinander um ein Mass F exzentrisch versetzt sein können. Die Aussenfläche 21 und die Innenfläche 22 sind durch Radien R4 bzw. R5 bestimmt, wobei der Radius R4 um ein einem vorbestimmten Spiel S entsprechendes Mass kleiner ist als der Radius R3 der Innenfläche 18 der Aussenschale 4. Wie aus der Fig. 1 hervorgeht, weist die Zwischenschale 5 eine nur im Polbereich der Aussenschale 4 an deren Innenfläche 18 anlegbare Stützpartie 21a auf und verläuft ausserhalb dieses Bereichs in einem gegen den äquatorialen Bereich der Aussenschale 4 hin zunehmenden Abstand von der Innenfläche 18, wobei das Spiel S entsprechende Relativbewegungen der äquatorialen Bereiche der mit der Innenschale 6 fest verbundenen Zwischenschale 5 und mindestens eines der Segmente 4a, 4b, 4c, 4d, 4e quer zur Hauptachse A zulässt. Entsprechend der Darstellung nach Fig. 1 kann die Innenschale 6 im äquatorialen Bereich ihrer Aussenfläche 25 mit einer Ringnut 23 ausgeführt sein, die zur Aufnahme einer von der Innenfläche 21 der Zwischenschale 5 nach innen abstehenden, in die Ringnut 23 einklinkbaren Kante 24 bestimmt ist.

Die beschriebene Anordnung ermöglicht auf einfache Weise und mit geringem Zeitaufwand eine genaue Positionierung der Gelenkpfanne 2 in dem entsprechend vorbereiteten Implantationsbereich, wobei die Aussenschale 4, die Zwischenschale 5 und die Innenschale 6 nacheinander eingesetzt oder vorgängig zu einer Implantationseinheit zusammengesetzt werden können. Durch die beschriebene kraft- und formschlüssige Verbindung zwischen der Aufsetzpartie 8 der Aussenschale 4 und der Stützpartie 12 der Innenschale 6 wird auf einfache Weise eine sichere, direkte Uebertragung der Hauptbelastungskraft B sowie eine sichere, winkelgetreue Führung des Gelenkkopfes 3 gewährleistet, wobei die Innenschale 6 im wesentlichen in einer bezüglich der Innenfläche 18 der Aussenschale 4 konzentrischen Grundstellung gehalten wird. Die um den Polbereich 17a bzw. um die Aufsetzpartie 8 verformbaren, nach Art von Biegefedern relativ zueinander beweglichen Segmente 4a, 4b, 4c, 4d, 4e der Aussenschale 4 gestatten entsprechende, unter Belastung auftretende Verformungen des Implantationsbereichs, z.B. örtliche Verengungen in einem Abschnitt und entsprechende Aufweitungen in einem anderen Abschnitt der die Aussenschale 4 umgebenden Ausnehmung des Knochengewebes 1 ,wobei diese Verformungen durch die Abmessungen der Zwischenschale 5 begrenzt werden und beim Auftreten von Aufweitungen ein örtliches Ablösen der Segmente vom Knochengewebe 1 verhindert wird.

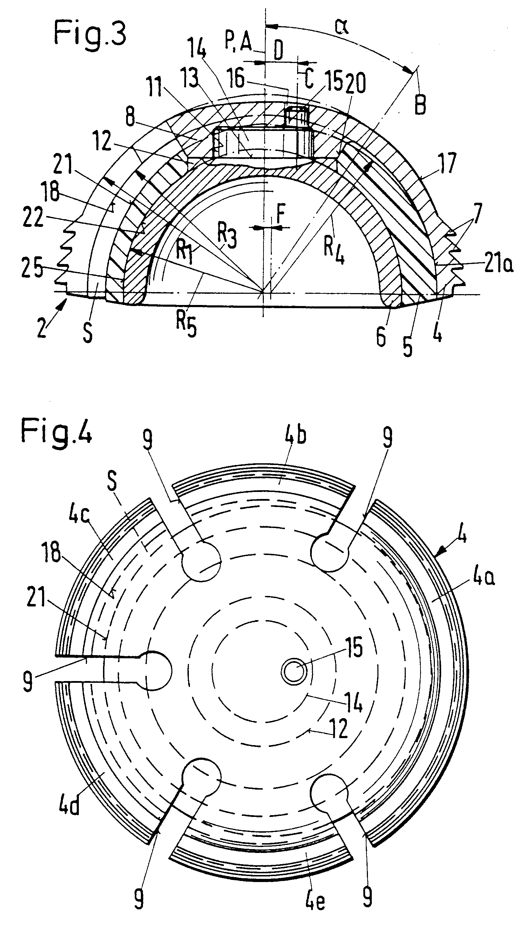

In den dargestellten Ausführungsbeispielen sind einander entsprechende Teile mit den gleichen Bezugszeichen versehen. Ensprechend der Darstellung nach den Fig. 3 und 4 kann die für die kraft- und formschlüssige Verbindung zwischen der Aussenschale 4 und der Innenschale 6 massgebende gemeinsame Hauptachse A mit der gegenüber der Wirkungslinie der Hauptbelastungskraft B versetzten Polachse P zusammenfallen. Entsprechend sind die Aufsetzpartie 8 der Aussenschale 4 und die Stützpartie 12 der Innenschale 6 je im betreffenden Polbereich ausgebildet. Bei dieser Ausführung sind die Krümmungsmittelpunkte der Aussenfläche 21 und der Innenfläche 22 der Zwischenschale 5 im Bereich einer gemeinsamen äquatorialen Ebene seitlich gegeneinander versetzt. Entsprechend liegt die Auflagefläche 21a der Zwischenschale 5 nur im äquatorialen Randbereich an der Innenfläche 18 der Aussenschale 4 an, so dass die Zwischenschale 5 mit der Aussenschale 4 einen in der Draufsicht (Fig. 4) sichelförmigen Spalt S begrenzt, der entsprechende Relativbewegungen der Zwischenschale 5 und der Segmente 4b, 4c, 4d, 4e der Aussenschale 4 zulässt, während zwischen dem Segment 4a und der Zwischenschale 5 eine relativ steife Verbindung besteht, welche eine sichere Uebertragung der Hauptbelastungskraft B zwischen der Innenschale 6 und der Aussenschale 4 gewährleistet.

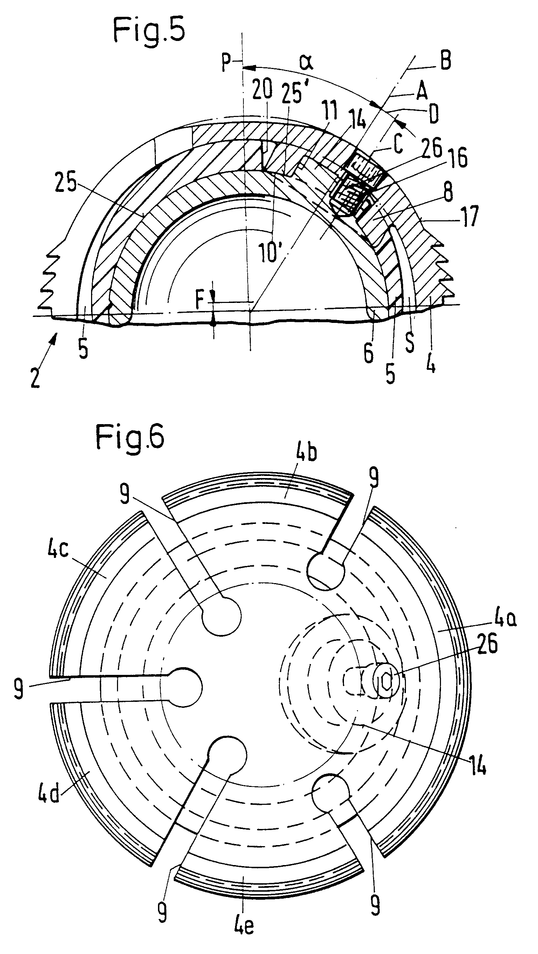

Die Gelenkpfanne 2 nach den Fig. 5 und 6 entspricht im wesentlichen der Ausführung nach den Fig. 1 und 2, wobei, im Unterschied zu dieser Ausführung, die Aufsetzpartie 8 mit einer an einen Teil 25' der sphärischen Aussenfläche 25 der Innenschale 6 anlegbaren, sphärischen Aufsetzfläche 10' ausgebildet ist. Entsprechend erübrigt sich die Ausbildung einer an die Aufsetzpartie 8 anzupassenden, zusätzlich zu bearbeitenden Stützpartie an der Innenschale 6, so dass diese, im Vergleich zu den vorstehend beschriebenen Ausführungen, in einer vereinfachten Bauweise ausgeführt werden kann, welche lediglich die Ausbildung eines einfachen zylindrischen Fortsatzes als Führungsteil 14 erfordert. Als Befestigungselement zur Sicherung der drehfesten Verbindung zwischen der Aussenschale 4 und der Innenschale 6 kann darstellungsgemäss eine von aussen her in den Führungsteil 14 einführbare, gegenüber der Hauptachse A exzentrisch versetzte Schraube 26 vorgesehen sein, welche die Aussenschale 4, die Zwischenschale 5 und die Innenschale 6 zu einer vorgängig zusammensetzbaren und als Ganzes implantierbaren Einheit verbindet. Wie aus der Fig. 5 hervorgeht, kann die Hauptachse A mit der Wirkungslinie der Hauptbelastungskraft B zusammenfallen.

Die Ausführung nach den Fig. 5 und 6 ergibt eine Gelenkpfanne 2 in einer konstruktiv einfachen und vorteilhaft kompakten Bauweise.

Zusammenfassend lässt sich die Erfindung wie folgt beschreiben:

Die Gelenkpfanne 2 enthält eine metallische Aussenschale 4, eine aus Kunststoff bestehende Zwischenschale 5 und eine in diese formschlüssig einsetzbare metallische Innenschale 6. Die Aussenschale 4 ist durch gegen ihren äquatorialen Randbereich hin offene Schlitze 9 in Segmente 4a, 4b, 4c, 4d, 4e unterteilt. Die Aussenschale 4 und die Innenschale 6 sind über eine Aufsetzpartie 8 in Richtung einer gemeinsamen Hauptachse A starr gegeneinander abgestützt und über ein gegenüber dieser Hauptachse A exzentrisch versetztes Befestigungselement 15 drehfest verbunden. Die Zwischenschale 5 ist mit einer die Aufsetzpartie 8 aufnehmenden Durchtrittsöffnung 20 sowie mit einer Aussenfläche 21 ausgeführt, welche eine zum Anlegen an einen Teil der Innenfläche 18 der Aussenschale 4 bestimmte Stützpartie 21a aufweist und ausserhalb dieser Stützpartie 21a mit dem restlichen Teil der Innenfläche 18 ein Spiel S begrenzt, welches entsprechende Relativbewegungen der die Innenschale 6 enthaltenden Zwischenschale 5 und mindestens eines der Segmente 4a, 4b, 4c, 4d, 4e der Aussenschale 4 quer zur Hauptachse A zulässt. Die implantierte Aussenschale 4 kann sich mit der sie umgebenden Knochenpartie unter Belastung elastisch verformen, während die sphärische Form der Innenschale 6 erhalten bleibt.

## Patentansprüche

1. Mehrteilige künstliche Gelenkpfanne, enthaltend eine in einen Implantationsbereich einsetzbare, im Knochengewebe (1) verankerbare metallische Aussenschale (4) mit beweglichen Segmenten (4a, 4b, 4c, 4d, 4e), die durch in Meridianen verlaufende Schlitze (9) begrenzt sind, eine in die Aussenschale (4) einsetzbare, aus einem Kunststoff bestehende Zwischenschale (5) und eine in diese formschlüssig einsetzbare metallische Innenschale (6) zur Aufnahme eines Gelenkkopfes (3), wobei die Aussenschale (4) und die Zwischenschale (5) sowie die Zwischenschale (5) und die Innenschale (6) je über eine zumindest annähernd halbkugelförmige Innen- bzw. Aussenfläche (18 bzw. 21, 22 bzw. 25) zusammenwirken, dadurch gekennzeichnet, dass die in der Aussenschale (4) ausgebildeten Segmente (4a, 4b, 4c, 4d, 4e) durch Eigenspannung im Knochengewebe verankerbar sind, dass die Aussenschale (4) und die Innenschale (6) in Richtung einer gemeinsamen, zumindest annähernd radialen Hauptachse (A) starr und über mindestens ein Befestigungselement (15) bezüglich dieser Hauptachse (A) drehfest miteinander verbunden sind, und dass die Aussenfläche (21) der Zwischenschale (5) eine zum Anlegen an einen Teil der Innenfläche (18) der Aussenschale (4) bestimmte Stützpartie (21a) aufweist und ausserhalb dieser Stützpartie (21) mit dem restlichen Teil der Innenfläche (1) der Aussenschale (4) ein vorbestimmtes Spiel (S) begrenzt, welches entsprechende Relativbewegungen der die Innenschale (6) enthaltenden Zwischenschale (5) und mindestens eines der Segmente (4a, 4b, 4c, 4d, 4e) der implantierten Aussenschale (4) quer zur gemeinsamen Hauptachse (A) zulässt.

2. Gelenkpfanne nach Anspruch 1, dadurch gekennzeichnet, dass die Aussenfläche (21) der Zwischenschale (5) mit einem Radius (R4) ausgeführt ist, der um ein dem vorbestimmten Spiel (S) entsprechendes Mass kleiner ist als der Radius (R3) der Innenfläche (18) der Aussenschale (4).

3. Gelenkpfanne nach Anspruch 1 oder 2, dadurch gekennzeichnet, dass die Aussenschale (4) im Bereich der Randpartie mit einer Anzahl von der Aussenfläche (17) abstehender, in das Knochengewebe (1) einführbarer Vorsprünge (7) ausgeführt ist, über welche die Segmente (4a, 4b, 4c, 4d, 4e) je im Implantationsbereich verankerbar sind.

4. Gelenkpfanne nach einem der vorangehenden Ansprüche, dadurch gekennzeichnet, dass die Aussenschale (4) mit einer Wandstärke ausgeführt ist, die im Biegebereich der Segmente (4a, 4b, 4c, 4d, 4e) eine elastische Verformung zur Primärverankerung der Segmente (4a, 4b, 4c, 4d, 4e) zulässt.

5. Gelenkpfanne nach einem der vorangehenden Ansprüche, dadurch gekennzeichnet, dass die Aussenschale (4) im Polbereich (17a) mit einer Wandstärke ausgeführt ist, die kleiner ist als die Wandstärke im äquatorialen Randbereich.

6. Gelenkpfanne nach einem der vorangehenden Ansprüche, dadurch gekennzeichnet, dass die Aussenfläche (21) und die Innenfläche (22) der Zwischenschale (5) zueinander exzentrisch ausgeführt sind.

7. Gelenkpfanne nach einem der vorangehenden Ansprüche, dadurch gekennzeichnet, dass die Aussenschale (4) im Bereich der gemeinsamen Hauptachse (A) mit einer nach innen abstehenden Aufsetzpartie (8) ausgeführt ist, die eine zum Zusammenführen mit der Innenschale (6) bestimmte Aufsetzfläche (10, 10') und eine nach innen offene Führungsbohrung (11) aufweist, dass die Zwischenschale (5) mit einer zur Aufnahme der Aufsetzpartie (8) bestimmten Durchtrittsöffnung (20) ausgeführt ist, und dass die Innenschale (6) mit einer an die Aufsetzfläche (10, 10') der Aufsetzpartie (8) anlegbaren Stützfläche (13, 25') und einem von dieser nach aussen abstehenden, in die Führungsbohrung (11) der Aufsetzpartie (8) einführbaren zapfenartigen Führungsteil (14) ausgeführt ist.

8. Gelenkpfanne nach Anspruch 7, dadurch gekennzeichnet, dass das die Aussenschale (4) und die Innenschale (5) verbindende Befestigungselement in Form eines am Führungsteil (14) oder an der Aussenschale (4) bezüglich der gemeinsamen Hauptachse (A) exzentrisch versetzt angeordneten Zentrierstifts (15, 26) ausgeführt ist, und dass am jeweils anderen Teil, Aussenschale (4) bzw. Führungsteil (14), eine entsprechend angeordnete Aussparung (16) zur Aufnahme des Zentrierstifts (15, 26) vorgesehen ist.

9. Gelenkpfanne nach einem der vorangehenden Ansprüche, dadurch gekennzeichnet, dass die gemeinsame Hauptachse (A) der Aussenschale (4) und der Innenschale (6) im wesentlichen der Wirkungslinie einer über die implantierte Gelenkpfanne zu übertragenden Hauptbelastungskraft (B) entspricht.

10. Hüftgelenkprothese mit einer Gelenkpfanne nach einem der Ansprüche 1 bis 9.

## Claims

1. A multi-part artificial acetabular cup, containing a metallic outer shell (4), insertable into an implantation area and anchorable in the bone tissue (1), having mobile segments (4a,4b,4c,4d,4e) which are limited by meridian extending slits (9), an intermediate shell (5) comprised of synthetic material, insertable into outer shell (4), and a metallic inner shell (6) which is form-lockingly insertable into same for incorporating a joint head (3), whereby outer shell (4) and intermediate shell (5) likewise intermediate shell (5) and inner shell (6) each co-operate through an at least approximately hemispherical-shaped inner or outer surface (18 or 21,22 or 25), characterised in that segments (4a,4b,4c,4d,4e) constructed in outer shell (4) are anchorable in the bone tissue by means of internal tension, that outer shell (4) and inner shell (6) are rigidly and rotation-fast connected to one another via at least one securing element (15) in the direction of a common, at least approximately radial main axis (A), and that outer surface (21) of intermediate shell (5) has a support part (21a) intended for laying on a part of inner surface (18), and beyond this support part (21), limits a predetermined play (S) with the remaining part of inner surface (1) of outer shell (4), which allows corresponding relative movement of intermediate shell (5) containing inner shell (6) and at least one of segments (4a,4b,4c,4d,4e) of implanted outer shell (4) transversely to the common main axis (A).

2. An acetabular cup according to claim 1, characterised in that outer surface (21) of intermediate shell (5) is arranged having a radius (R4) which, corresponding to the predetermined play (S) about a mass is smaller than the radius (R3) of inner surface (18) of outer shell (4).

3. An acetabular cup according to claims 1 or 2, characterised in that outer shell (4) is provided with a number of projections (7) arranged in the edge part area which are insertable in the bone tissue (1) and spaced from outer surface (17), through which segments (4a,4b,4c,4d,4e) are each anchorable in the implantation area.

4. An acetabular cup according to any of the previous claims, characterised in that outer shell (4) is provided with a wall thickness which permits a flexible distortion for the primary anchorage of segments (4a,4b,4c,4d,4e) in the bending area of segments (4a,4b,4c,4d,4e).

5. An acetabular cup according to any of the previous claims, characterised in that outer shell (4) has a wall thickness in the pole area (17a) which is smaller than the wall thickness in the equatorial edge area.

6. An acetabular cup according to any of the previous claims, characterised in that outer surface (21) and inner surface (22) of the intermediate shell (5) are arranged eccentrically to one another.

7. An acetabular cup according to any of the previous claims, characterised in that outer shell (4) is provided with an inwardly spaced inset part (8) in the area of the common axis (A), which has an inset surface (10,10') intended for combining with inner shell (6), and an inwardly opening guide bore (11), that intermediate shell (5) is provided with a through orifice (20) intended to incorporate inset part (8), and that inner shell (6) is provided with a support surface (13,25') which can be laid on the inset surface (10,10') of inset part (8) and a peg-like guide part (14) which is outwardly spaced from this and which is insertable into guide bore (11) of inset part (8).

8. An acetabular cup according to claim 7, characterised in that the securing element joining outer shell (4) and inner shell (5) is provided in the form of a guide part (14) or centring pin (15) arranged on the outer shell (4), eccentrically displaced with regard to the common main axis (A), and that on each other part, outer shell (4) or guide part (14), a correspondingly arranged recess (16) is provided to incorporate centring pin (15,26).

9. An acetabular cup according to any of the previous claims, characterised in that the common main axis (A) of outer shell (4) and inner shell (6) essentially corresponds to the line of action of a main load force (B) to be transferred through the implanted acetabular cup.

10. A hip joint prosthesis with an acetabular cup according to any of claims 1-9.

## Revendications

1. Acétabule artificiel en plusieurs parties, comportant une coque extérieure métallique (4) pouvant être mise en place dans une zone d'implantation, pouvant être ancrée dans le tissu osseux (1), avec des segments mobiles (4a, 4b, 4c, 4d, 4e), qui sont délimités par des fentes (9) s'étendant en méridiennes, une coque intermédiaire (5) pouvant être placée dans la coque extérieure (4) réalisée en une matière synthétique et une coque intérieure métallique (6) pouvant être placée par concordance des formes dans celle-ci pour la réception d'une tête d'articulation (3), la coque extérieure (4) et la coque intermédiaire (5) ainsi que la coque intermédiaire (5) et la coque intérieure (6) coopérant respectivement par une surface intérieure respectivement extérieure au moins approximativement semi-sphérique (18, 21 ; 22, 25) caractérisé en ce que les segments (4a, 4b, 4c, 4d, 4e) réalisés dans la coque extérieure (4) peuvent être ancrés par contrainte propre dans le tissu osseux, que la coque extérieure (4) et la coque intérieure (6), en direction d'un axe principal (A) commun, au moins approximativement radial, sont reliés rigidement et par au moins un élément de fixation (15) relativement à cet axe principal (A) d'une manière immobile en rotation, et en ce que la surface extérieure (21) de la coque intermédiaire (5) présente une partie d'appui (21a) prévue pour l'application à une partie de la surface intérieure (18) de la coque extérieure (4) et délimite à l'extérieur de cette partie d'appui (21) avec la partie restante de la surface intérieure (1) de la coque extérieure (4) un jeu prédéterminé (S) qui permet des mouvements relatifs correspondants de la coque intermédiaire (5) contenant la coque intérieure (6) et au moins l'un des segments (4a, 4b, 4c, 4d, 4e) de la coque extérieure implantée (4) transversalement à l'axe principal commun (A).

2. Acétabule selon la revendication 1, caractérisé en ce que la surface extérieure (21) de la coque intermédiaire (5) est réalisée avec un rayon (R4) qui est plus petit d'une dimension correspondant au jeu prédéterminé (S) que le rayon (R3) de la surface intérieure (18) de la coque extérieure (4).

3. Acétabule selon la revendication 1 ou 2, caractérisé en ce que la coque extérieure (4) est réalisée au voisinage de la partie de bord avec un certain nombre de saillies (7) dépassant de la surface extérieure (17), pouvant être insérées dans le tissu osseux (1) par lesquelles les segments (4a, 4b, 4c, 4d, 4e) peuvent être ancrés respectivement dans la zone d'implantation.

4. Acétabule selon l'une des revendications précédentes, caractérisé en ce que la coque extérieure (4) est réalisée avec une épaisseur de paroi qui permet dans la zone de flexion des segments (4a, 4b, 4c, 4d, 4e) une déformation élastique pour l'ancrage primaire des segments (4a, 4b, 4c, 4d, 4e).

5. Acétabule selon l'une des revendications précédentes, caractérisé en ce que la coque extérieure (4) est réalisée dans la zone polaire (17a) en une épaisseur de paroi qui est plus petite que l'épaisseur de paroi dans la zone de bord équatoriale.

6. Acétabule selon l'une des revendications précédentes, caractérisé en ce que la surface extérieure (21) et la surface intérieure (22) de la coque intermédiaire (5) sont réalisées d'une manière excentrique l'une vers l'autre.

7. Acétabule selon l'une des revendications précédentes, caractérisé en ce que la coque extérieure (4) est réalisée au voisinage de l'axe principal commun (A) avec une partie d'application (8) faisant saillie vers l'intérieur qui présente une surface d'application (10, 10') prévue pour la réunion avec la coque intérieure (6) et un percage de guidage (11) ouvert vers l'intérieur, que la coque intermédiaire (5) est réalisée avec une ouverture traversante (20) destinée à recevoir la partie d'application (8) et en ce que la coque intérieure (6) est réalisée avec une surface d'appui (13, 25') applicable à la surface d'application (10, 10') de la partie d'application (8) et avec une partie de guidage en forme d'ergot (14) faisant saillie vers l'extérieur à partir de celle-ci, insérable dans le perçage de guidage (11) de la partie d'application (8).

8. Acétabule selon la revendication 7, caractérisé en ce que l'élément de fixation reliant la coque extérieure (4) et la coque intérieure (5) est réalisé sous la forme d'une tige de centrage (15, 26) disposée à la pièce de guidage (14) ou à la coque extérieure (4) selon un décalage excentrique relativement à l'axe principal commun (A) et en ce qu'il est prévu à l'autre partie respective, la coque extérieure (4) respectivement la pièce de guidage (14), un évidemment (16) disposé de manière correspondante pour recevoir la tige de centrage (15, 26).

9. Acétabule selon l'une des revendications précédentes, caractérisé en ce que l'axe principal commun (A) de la coque extérieure (4) et de la coque intérieure (6) correspond essentiellement à la ligne d'action d'une force de charge principale (B) à transférer par l'acétabule implanté.

10. Prothèse d'articulation de la hanche avec un acétabule selon l'une des revendications 1 à 9.
